# EUROPÄISCHE PATENTANMELDUNG

(11) **EP 1 256 897 A2**
(43) Veröffentlichungstag der Anmeldung: **13.11.2002**
(21) Anmeldenummer: 02009687.1
(22) Anmeldetag: 29.04.2002
(51) Int. Cl.: G06F 19/00

(54) **Verfahren zum Überwachen des Therapieverlaufs eines zu therapierenden Patienten**

(30) Priorität: 10.05.2001 DE 10122778
(71) Anmelder: SIEMENS AKTIENGESELLSCHAFT, 80333 München (DE)
(72) Erfinder: Schmidt, Volker, Dr., 91054 Erlangen (DE); Schneider, Siegfried, Dr., 91056 Erlangen (DE); Tiffe, Sven, 91052 Erlangen (DE)

(57) **Zusammenfassung**

Verfahren zum Überwachen des Therapieverlaufs eines zu therapierenden Patienten, bei dem wenigstens ein eine therapeutische und/oder diagnostische Maßnahme erfordernder Informationswert, der ein direktes oder indirektes Maß für den Therapieverlauf ist, kontinuierlich oder in Zeitintervallen aufgenommen wird, und dem wenigstens ein mit ihm oder mit einem basierend auf dem Informationswert ermittelten Rechenwert zu vergleichender Vergleichwert zugeordnet ist, wobei bei einer im Rahmen des Vergleichs festgestellten gegebenen oder sich einstellenden unzulässigen Abweichung des Informationswerts oder des Rechenwerts vom Vergleichswert eine Alarminformation ausgegeben wird, und wobei der Vergleichswert in Abhängigkeit des Therapieverlaufs variiert und dem Therapieverlauf angepasst wird, wobei die Anpassung des wenigstens einen Vergleichswerts automatisch oder durch eine autorisierte Person oder Stelle erfolgt, wobei die Anpassung in vorgegebenen Zeitabständen oder in variablen Zeitabständen und abhängig vom aufgenommenen Informationswert oder dem ermittelten Rechenwert erfolgt, und wobei bei einer durch eine Person oder Stelle erfolgenden Anpassung die Person oder Stelle automatisch auf das Erfordernis der Anpassung hingewiesen wird.

## Beschreibung

Die Erfindung betrifft ein Verfahren zum Überwachen des Therapieverlaufs eines zu therapierenden Patienten, bei dem wenigstens ein eine therapeutische und/oder diagnostische Maßnahme erfordernder Informationswert, der ein direktes oder indirektes Maß für den Therapieverlauf ist, kontinuierlich oder in Zeitintervallen aufgenommen wird, und dem wenigstens ein mit ihm oder mit einem basierend auf dem Informationswert ermittelten Rechenwert zu vergleichender Vergleichwert zugeordnet ist, wobei bei einer im Rahmen des Vergleichs festgestellten gegebenen oder sich einstellenden unzulässigen Abweichung des Informationswerts oder des Rechenwerts vom Vergleichswert eine Alarminformation ausgegeben wird, und wobei der Vergleichswert in Abhängigkeit des Therapieverlaufs variiert und dem Therapieverlauf angepasst wird.

Liegt ein oder liegen diverse gesundheitsrelevante Werte oder Parameter eines Patienten außerhalb eines definierten Normalbereichs, so wird versucht, diesen Wert oder diesen Parameter des Patienten durch eine geeignete, gezielte Therapie auf einen in der Regel diesem Normalbereich entsprechenden Zielbereich einzustellen. Beispielsweise leidet der Patient an Bluthochdruck. Der gesundheitsrelevante Wert wäre in diesem Fall der auf einfache Weise messbare Blutdruck, der gesenkt werden muss, so dass er in einem gewünschten Zielbereich liegt. Diese Phase, die in der Regel Einstellungsphase genannt wird, kann zeitlich begrenzt sein, wobei zum Ende der Einstellungsphase der oder die überwachten Werte den Zielbereich spätestens erreicht haben sollen. Ist der Patient eingestellt so werden die Werte in der nachfolgenden Erhaltungsphase weiterhin überwacht.

Verlassen die Werte während der Einstellungs- oder Erhaltungsphase einen definierten Wertebereich, was durch Vergleichen des aufgenommenen Werts oder Parameters mit den Wertebereich definierenden Vergleichswerten erfasst werden kann, so kann eine Änderung der Behandlungsmethode sowie weitere Maßnahmen, wie z.B. Diagnostik etc. notwendig werden. Ändert sich der aufgenommene Wert zum Positiven, stellt sich also eine Verbesserung und damit ein therapeutisch positiver Effekt ein, so ist dies für den Patienten insoweit nicht nachteilig. Ändert sich jedoch der Wert in negativer Weise, so muss eine verantwortliche Person oder Stelle, z.B. ein Arzt hiervon in Kenntnis gesetzt und alarmiert werden. Es muss also ein Alarmsignal ausgegeben werden, das auf den negativen Zustand hinweist. Zugeordnet sind im Stand der Technik starre Alarmgrenzen. Dies führt aber häufig zu Fehlalarmen oder zu fehlenden Alarmen, wenn ein instabiles System sich auf einen stabilen Wert einstellt. Beispielsweise kommt ein derartiges Verfahren oder ein derartiges System in der Intensivmedizin der stationären Behandlung zum Einsatz, wo teilweise eine automatische Überwachung kritischer Vitalparameter stattfindet.

Aus US 5,749,907 A ist ein Verfahren mit den eingangs genannten Merkmalen bekannt. Mit ihm können Abweichungen des aufgenommenen Informationswerts von einem Vergleichswert erfasst und der vor Ort befindliche Arzt während eines Besuches alarmiert werden, wobei der Arzt die Möglichkeit hat, innerhalb des Vergleichs auch zeitlich früher aufgenommene Informationswerte zu berücksichtigen. Ferner hat der Arzt die Möglichkeit, den Vergleichswert zu verändern, wenn er beim Patienten ist oder war.

Der Erfindung liegt das Problem zugrunde, ein im Hinblick auf eine sicherere und zuverlässigere Alarmierung verbessertes Verfahren anzugeben.

Zur Lösung dieses Problems ist bei einem Verfahren der eingangs genannten Art vorgesehen, dass die Anpassung des wenigstens einen Vergleichswerts automatisch oder durch eine autorisierte Person oder Stelle erfolgt, wobei die Anpassung in vorgegebenen Zeitabständen oder in variablen Zeitabständen und abhängig vom aufgenommenen Informationswert oder dem ermittelten Rechenwert erfolgt, und wobei bei einer durch eine Person oder Stelle erfolgenden Anpassung die Person oder Stelle automatisch auf das Erfordernis der Anpassung hingewiesen wird.

Beim erfindungsgemäßen Verfahren wird wenigstens ein Informationswert aufgenommen. Dieser kann ein direktes Maß für den Therapieverlauf sein. Beispielsweise kann als Informationswert der Blutdruck gemessen werden, oder ein die Blutgerinnung beschreibender Wert, der Insulinspiegel oder dergleichen. Der Informationswert kann aber auch ein indirektes Maß für den Therapieverlauf sein. Als Beispiel ist hier z.B. bei einem Asthmatiker die Ausatemgeschwindigkeit oder das Ausatemvolumen zu nennen, anhand welchen Rückschlüsse auf den Bronchenzustand gezogen werden können.

Diesem Informationswert, der kontinuierlich oder in beliebigen gleichmäßigen oder ungleichmäßigen Zeitintervallen aufgenommen werden kann, kann entweder unmittelbar ein Vergleichswert zugeordnet sein, der eine Grenze bildet, bezüglich welcher der Informationswert in einem bestimmten Verhältnis liegen muss. Alternativ besteht die Möglichkeit, anhand des Informationswerts einen Rechenwert zu ermitteln, diesen also weiterzuverarbeiten, z.B. mit weiteren aufgenommenen, anderen und für die Therapie relevanten Informationswerten zu verknüpfen, oder aber mehrere zeitlich unterschiedlich aufgenommene Informationswerte miteinander zu verknüpfen. Auch dieser Rechenwert ist mit dem Vergleichswert zu vergleichen, um eine zulässige oder unzulässige Abweichung oder ein zulässiges oder unzulässiges Verhältnis des Rechenwerts zum Vergleichswert zu ermitteln.

Liegt der Informations- oder der Rechenwert in einem zulässigen Verhältnis oder mit einer zulässigen Abweichung bezüglich des Vergleichswerts, so ist die Gabe einer Alarminformation nicht erforderlich, da der Therapieverlauf positiv, also förderlich und wie angestrebt ist. Ergibt der Vergleich jedoch eine gegebene oder eine sich aufgrund einer anhand des Rechenwerts erstellten Trendanalyse in Kürze einstellende unzulässige Abweichung vom Vergleichswert, so muss eine Alarminformation an eine zuständige Person oder Stelle ausgegeben werden.

Die besonders vorteilhafte Ausprägung des erfindungsgemäßen Verfahrens sieht nun vor, dass der Vergleichswert nicht starr ist, sondern variabel in Abhängigkeit des Therapieverlaufs gewählt und dem Therapieverlauf angepasst werden kann. Befindet sich der Patient also beispielsweise in der Einstellungsphase, in welcher der hohe Blutdruck auf einen normalen Blutdruck gesenkt werden soll, so wird dem jeweiligen Informations- oder Rechenwert zu Beginn der Behandlung ein hoher Vergleichswert, wenn also auch der Informationswert oder der Rechenwert entsprechend hoch sind, zugeordnet. Mit zunehmendem Behandlungsverlauf wird sich bei positiv verlaufender Therapie der Informations- oder Rechenwert senken. Dies wird mit dem zugeordneten Vergleichswert nachvollzogen, der ebenfalls entsprechend gesenkt wird. Für den Fall, dass nun unvorhergesehenerweise der Blutdruck wieder ansteigt und über dem angepassten Vergleichswert liegt, wird ein Alarmsignal gegeben, da dieser Zustand als Alarmzustand erkannt werden kann aufgrund der Anpassung des Vergleichswerts. Wäre eine starre Vergleichswertgrenze definiert, die zwangsläufig in Anbetracht des hohen Ausgangsblutdruckwerts definiert wäre, so würde in dem geschilderten Fall kein Alarmsignal gegeben werden, da bei einer Erhöhung des bereits therapeutisch abgesenkten Blutdrucks die starre Vergleichs- oder Alarmgrenze nicht überschritten worden wäre.

Der Vergleichswert kann gemäß dem erfindungsgemäßen Verfahren beliebig in Anbetracht des Therapieverlaufs variiert und angepasst werden, d.h., er wird z.B. bei kontinuierlich einstellender Blutdrucksenkung ebenfalls kontinuierlich schritt- oder stufenweise abgesenkt. Auf diese Weise ist es also mit besonderem Vorteil möglich, das Monitoring des Wertes und die Gabe der Alarminformation miteinander so zu korrelieren, dass tatsächlich auch der Istzustand berücksichtigt wird.

Neben der beschriebenen Anwendung des Verfahrens im Rahmen der Blutdruckmessung kann dieses erfindungsgemäße Verfahren natürlich beliebig angewandt werden. Als weiteres Beispiel ist z.B. die Überwachung der Blutgerinnungswerte zu nennen. Hierbei ermittelt der Patient - wie dies auch im Falle des Blutdruckmessens der Fall sein kann - selbst und zu Hause den jeweiligen Informationswert, hier also den Blutgerinnungswert, der dann entsprechend wie beschrieben verarbeitet wird. Gleiches gilt betreffend einen an Diabetes leidenden Patienten, der z.B. selbst in Zeitintervallen seinen Blutzuckerwert und sein Gewicht erfasst, wobei bei diesem Beispiel zwei Informationswerte in den Vergleich mit eingehen. Auch sind weitere Kombinationen denkbar, z.B. bei einem Diabetiker mit Bluthochdruck. Hier sind einerseits die diabetesbezogenen Informationswerte zu erfassen, zum anderen auch der Blutdruckwert, da sich die relevanten Parameter aus therapeutischer Sicht mitunter nachteilig beeinflussen können und infolgedessen beide im Rahmen der Beurteilung, ob eine Vergleichswertanpassung zweckmäßig und erforderlich ist, beachtet werden müssen. Z.B. wirkt sich in einem solchen Fall ein gegen Bluthochdruck gegebener Betablocker nachteilig auf den Insulinspiegel aus.

Die Anpassung des wenigstens einen Vergleichswerts kann erfindungsgemäß automatisch durch eine geeignete Anpasseinrichtung des Kontrollsystems erfolgen. Bei dieser Anpasseinrichtung handelt es sich z.B. um eine zentrale Recheneinrichtung, die sämtliche relevanten Informationswerte erhält, und in der die relevanten Vergleichswerte abgelegt sind, und die sämtliche Vergleichs- und sonstigen Rechenoperationen sowie die Ausgabe entsprechender Alarminformationen etc. steuert. Alternativ dazu besteht auch die Möglichkeit, dass eine autorisierte Person oder eine autorisierte Stelle, z.B. ein Arzt oder die zuständigen Personen innerhalb einer Arztpraxis oder einer Klinik, diese Anpassung vornehmen, wenn ihnen die Vergleichsergebnisse vorliegen. Dabei ist es zweckmäßig, wenn bei einer manuellen, durch eine Person oder Stelle erfolgenden Anpassung die Person oder die Stelle automatisch von der zentralen Systemsteuerung auf das Erfordernis der Anpassung hingewiesen werden.

Die Anpassung des oder der Vergleichswerte kann in vorgegebenen Zeitabständen oder in variablen Zeitabständen erfolgen. Eine zeitlich vorgegebene Anpassung kann z.B. dergestalt ablaufen, dass nach Ermittlung des Erfordernisses der Anpassung diese zu einem bestimmten Zeitpunkt erfolgt, eine zeitlich variable Anpassung kann zu jedem beliebigen Zeitpunkt z.B. unmittelbar nach Ermittlung des Erfordernisses der Anpassung erfolgen. Welcher Anpassungsmodus gewählt wird, kann abhängig vom aufgenommenen Informationswert oder dem ermittelten Rechenwert sein.

Verwendet werden kann zur Durchführung des erfindungsgemäßen Verfahrens z.B. ein Monitoring-System, wie es aus der europäischen Patentanmeldung EP 00 124 669 bekannt ist. Es wird ausdrücklich Bezug auf sämtliche Merkmale und den gesamten Offenbarungsgehalt dieser Patentanmeldung genommen, der hiermit vollständig in den Offenbarungsgehalt der vorliegenden Anmeldung eingebunden wird und zum Teil dieser vorliegenden Erfindungsoffenbarung gemacht wird.

Da in der Regel ein kontrollierter Wert oder Parameter innerhalb bestimmter Grenzen liegen sollte, ist es vorteilhaft, wenn dem Informationswert oder dem Rechenwert ein oberer und ein unterer Vergleichswert zugeordnet werden, wobei der Informationswert oder der Rechenwert im Rahmen des Vergleichs mit beiden verglichen wird um zu überprüfen, ob er sich im definierten Intervall befindet oder nicht. Eine unzulässige Abweichung kann dann gegeben sein, wenn der Informationswert oder der Rechenwert oberhalb des oberen oder unterhalb des unteren Vergleichswertes liegt.

Neben dem Vergleich eines einzelnen aufgenommenen Informationswertes oder eines einzeln ermittelten Rechenwerts kann es für das erfindungsgemäße Verfahren auch vorteilhaft sein, eine sich in naher Zukunft voraussichtlich einstellende unzulässige Abweichung und anhand eines Vergleichs eines sich durch Analyse zeitlich früher aufgenommene Informationswerte oder ermittelter Rechenwerte ermittelten Therapietrends mit dem wenigstens einen Vergleichswert durchzuführen. D.h., es erfolgt hier eine Trendanalyse, die einen bestimmten, zukünftigen Gang des Therapieverlaufs indiziert, wobei im Rahmen des Vergleichs dieser indizierte Verlauf berücksichtigt wird. Zeigt sich also beispielsweise im Rahmen eines Blutdruck-Monitorings, dass innerhalb der letzten vier oder fünf gemessenen Blutdruckwerte ein ansteigender Trend vorliegt, so kann, wenn beispielsweise der zuletzt aufgenommene Wert nahe der z.B. oberen Vergleichswertgrenze, jedoch noch im zulässigen Bereich liegt, dennoch eine Alarminformation gegeben werden, da damit zu rechnen ist, dass der nächstaufgenommene Wert entweder den Trend fortsetzt oder selbst bereits über dem Vergleichswert liegt. Man nutzt also so den prognostizierbaren Therapieverlauf, um vorauseilend eine etwaige Alarminformation aufzugeben.

Wie beschrieben ist es denkbar, lediglich einen Informationswert oder einen darauf gestützt ermittelten Rechenwert im Rahmen des Vergleichs und der Vergleichswertanpassung zu berücksichtigen. Es kann jedoch auch vorgesehen sein, die Anpassung in Abhängigkeit mehrerer unterschiedlicher aufgenommener Informationswerte oder ermittelter Rechenwerte vorzunehmen, denen dann jeweils wenigstens ein eigener Vergleichswert zugeordnet ist. Es kann beispielsweise der Fall auftreten, dass durch die Gabe eines bestimmten, der Behandlung eines bestimmten Symptoms dienenden Medikaments ein negativer Effekt bezüglich eines anderen Symptoms, das ebenfalls zu therapieren ist, auftritt. Beispielsweise stellt sich bei Gabe eines der Blutdrucksenkung dienenden Betablockers ein schlechterer Insulinwert ein, d.h., die beiden Werte sind insoweit miteinander korreliert. Für die Gabe des Alarmsignals bzw. auch für die Anpassung z.B. des Insulin-Vergleichswerts ist diese Kenntnis, dass nämlich ein Betablocker gegeben wurde, relevant, da hierdurch erfasst werden kann, dass die Verschlechterung des Insulin-Werts auf die Betablocker-Gabe zurückzuführen ist und nicht auf eine generelle Verschlechterung der Konstitution des Patienten.

Die Anpassung selbst kann in Abhängigkeit des Vergleichs des Informationswerts oder des Rechenwerts mit dem wenigstens einen Vergleichswert dergestalt erfolgen, dass bei einem zulässigen Abweichen von dem Vergleichswert die Anpassung vorgenommen wird. Liegt also beispielsweise der aufgenommene Informationswert oder der daraus basierende Rechenwert, der in der Einstellungsphase sinken soll, innerhalb eines bestimmten Werteintervalls um die untere Grenze des über zwei Vergleichswerte definierten Vergleichsintervalls, so weicht einerseits der Informationswert in zulässiger Weise von beiden Vergleichswerten ab (er ist kleiner als der obere und größer als der untere Vergleichswert), zum anderen nähert er sich der unteren Intervallgrenze, was indiziert, dass eine Anpassung unter Absenkung des definierten Vergleichswerteintervalls zu niedrigeren Intervallwerten erforderlich ist. Alternativ kann die Anpassung auch in Abhängigkeit eines aus der Aufnahme zeitlich früherer Informationswerte oder früher ermittelter Rechenwerte ermittelten Therapietrends erfolgen. In jedem Fall muss sichergestellt sein, dass die Anpassung bezogen auf den Zustand des Patienten gerechtfertigt ist.

Weiterhin kann vorgesehen sein, dass jedem Vergleichswert ein Tolerenzwertebereich zugeordnet ist. Hierdurch soll sichergestellt werden, dass auch eine geringfügige Über- oder Unterschreitung eines Vergleichswerts, die streng genommen zur Abgabe einer Alarminformation führen würde, nicht zur Ausgabe der Information führt, sondern als tolerierbare kurzzeitige unzulässige Abweichung klassifiziert wird. Selbstverständlich muss sichergestellt sein, dass eine dauerhafte Abweichung ohne Alarminformationsgabe ausgeschlossen wird. Beispielsweise kann eine definierte Anzahl von unzulässigen Abweichungen bestimmt werden, ohne dass ein Alarmsignal ausgegeben wird. Bei einer erneuten unzulässigen Abweichung und einer Überschreitung der definierten Anzahl erfolgt dann sofort die Alarminformationsgabe.

In Weiterbildung des Erfindungsgedankens kann vorgesehen sein, dass der verantwortlichen Person oder Stelle eine Information gegeben wird, wenn sich innerhalb eines vorbestimmten Zeitintervalls ein angestrebter, durch die Aufnahme des Informationswerts oder die Ermittlung des Rechenwerts erfassbarer Therapieerfolg nicht einstellt. Hierdurch soll vermieden werden, dass das Monitoring-System kontinuierlich weiterarbeitet und der Patient kontinuierlich auf ein und dieselbe Weise therapiert wird, ohne dass sich der gewünschte Therapieerfolgt einstellt oder das Therapieziel erreicht oder sich diesem angenähert wird. Durch diese Erfindungsausgestaltung wird sichergestellt, dass dann, wenn sich ein bestimmter Zielwert innerhalb eines bestimmten Zeitintervalls nicht einstellt, eine Information an den behandelnden Arzt oder dergleichen gegeben wird. Dies kann z.B. auch anhand der Vergleichswerte erfolgen. Wenn dem Informationswert oder dem Rechenwert ein Vergleichswerteintervall, über einen oberen und einen unteren Vergleichswert definiert, zugeordnet wird, so kann z.B. bei einer gezielten Absenkung des jeweiligen zu therapierenden Parameters oder Symptoms, z.B. des Blutdrucks, dem unteren Vergleichswert ein Zeitintervallwert zugeordnet werden, wobei die Alarminformation dann ausgegeben wird, wenn der Informationswert oder der Rechenwert innerhalb des über diesen Zeitintervallwerts definierten Zeitintervalls keine zulässige Abweichung vom oder Annäherung an den Vergleichswert aufweist. Als zulässige Abweichung wird in dem Fall, dass der Blutdruckwert kontinuierlich sinken muss, ein hinreichendes Annähern an diesen Endvergleichswert oder eine Unterschreitung desselben definiert. Hierdurch wird vorteilhaft sichergestellt, dass das Monitoring-System nicht starr einen sich kontinuierlich nicht oder nur unwesentlich ändernden Zustand überwacht, sondern dass es quasi selbstkontrollierend den Therapieerfolg überwacht und das Erreichen des Therapieziels unterstützt.

Besonders zweckmäßig ist es zur Erreichung dieses erfindungsgemäßen Ziels, wenn der Person oder der Stelle mit der Gabe der Information über die Nichterreichung des Therapieziels weitere therapeutisch oder diagnostisch relevante Informationen gegeben werden, die zweckmäßigerweise aus einer systemimmanenten Wissensdatenbank gelesen werden. Diese Information kann z.B. alternative Therapie- oder Behandlungsmethode als die momentan angewandte und offensichtlich nicht zum Erfolg führende Therapiemethode beinhalten oder sonstige therapeutisch oder diagnostisch relevante Informationen. Selbstverständlich können derartige Informationen ganz generell während der laufenden Überwachung gegeben werden. Zu diesem Zweck ist die Nutzung einer systemimmanenten Wissensdatenbank zweckmäßig, z.B. in Form des "Arden Syntax for Medical Logic Systems". Derartige Wissensdatenbanken sind bekannt, ihre Einbindung in das vorliegende Verfahren ist zur weiteren Verbesserung des Überwachungsverfahrens und -systems im Hinblick auf eine optimale und möglichst zügige Erreichung des Behandlungsziels von Vorteil.

Wie bereits beschrieben umfasst das System, das zur Durchführung des erfindungsgemäßen Verfahrens geeignet ist, zweckmäßigerweise eine Rechen- oder Systemzentrale oder Systemsteuerung, die die relevanten Informationswerte erhält, gegebenenfalls die Rechenwerte ermittelt oder Trendanalysen durchführt sowie sämtliche sonstigen Vergleichs- oder Rechenoperationen durchführt und die Ausgabe relevanter Informationen, sei es in Form einer Alarminformation oder einer Information aus einer Wissensdatenbank oder dergleichen steuert. Auch die Anpassung des wenigstens einen Vergleichswerts wird über diese Rechen- oder Systemzentrale vorgenommen. In diesem Zusammenhang ist es generell zweckmäßig, wenn ein Expertensystem insbesondere zur Durchführung der beschriebenen Schritte verwendet wird, das hierbei unterstützend arbeitet. Dieses Expertensystem bezieht seine Daten aus einer Datenbank oder einem Netzwerk und generiert in Abhängigkeit des zu therapierenden Patienten sowie des zu therapierenden Symptoms und des Therapieverlaufs entsprechende Informationen, die im Bedarfsfall ausgegeben werden.

Wie bereits beschrieben kann das Verfahren mit einem beliebigen Monitoring-System durchgeführt werden, das die Aufnahme des oder der relevanten Informationswerte sowie die entsprechende Auswertung derselben zulässt. Es kann sich um ein zentrales oder dezentrales System handeln, denkbar sind unterschiedliche Systemvariationen. Z.B. kann die Informationswerterfassung patientenseitig zu Hause erfolgen, die Übermittlung der Informationswerte kann über einen patientenseitigen Rechner an eine Rechen- oder Systemzentrale erfolgen, die dann die entsprechenden Auswertungen und Schritte vornimmt. Auch kann die Auswertung patientenseitig vorgenommen werden, wobei dann die etwaige Alarmsignalgabe über ein Kommunikationsnetz, z.B. über den patientenseitigen Rechner an den Empfänger gesteuert erfolgen. Denkbar ist auch die Verwendung eines Handys zur Informationswertübertragung etc. In diesem Zusammenhang wird nochmals auf die eingangs genannte europäische Patentanmeldung EP 00 124 669 verwiesen.

Neben dem erfindungsgemäßen Verfahren betrifft die Erfindung ferner ein System zum Überwachen des Therapieverlaufs eines zu therapierenden Patienten, das zur Durchführung des beschriebenen Verfahrens geeignet ist.

Weitere Vorteile, Merkmale und Einzelheiten der Erfindung ergeben sich aus dem im folgenden beschriebenen Ausführungsbeispiel sowie anhand der Zeichnungen. Dabei zeigen:
- Fig. 1: ein Diagramm zur Darstellung des Grundprinzips des erfindungsgemäßen Verfahrens, und
- Fig. 2: eine Prinzipskizze eines Systems geeignet zur Durchführung des Verfahrens.

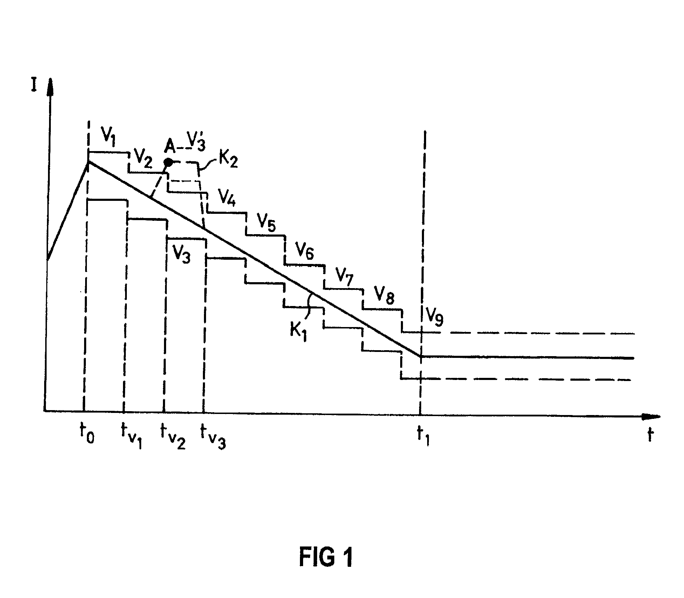

Fig. 1 zeigt in Form eines Diagramms das grundsätzliche Ablaufschema des erfindungsgemäßen Verfahrens. Längs der Abszisse ist die Zeit, längs der Ordinate der Informationswert I dargestellt. Ersichtlich steigt der Informationswert I zu Beginn der Kurve stark an, d.h., es stellt sich ein pathologisches Symptom ein. Z.B. nimmt der Blutdruck des Patienten deutlich zu. Der Blutdruck wird beispielsweise vom Patienten zu Hause unter Verwendung eines geeigneten Blutdruckmessgeräts überwacht und die Messwerte werden z.B. über einen patientenseitigen Eingaberechner an einen externen zentralen Rechner oder eine Systemsteuereinheit übertragen. In diesen zentralen Rechner oder diese Systemsteuereinheit wird der Informationswert im gezeigten Beispiel direkt berücksichtigt, ohne vorher in einen Rechenwert umgerechnet zu werden. Seitens der Steuereinheit ist dem Informationswert I im Zeitpunkt t₀, der den Beginn der Einstellungsphase definiert, also den Beginn der Therapie zur Absenkung des Blutdrucks, ein Vergleichswertepaar zugeordnet, wobei ein oberer und ein unterer Vergleichswert ein Vergleichswerteintervall bezogen auf den Informationswert definieren. Der Patient misst nun in kontinuierlichen oder diskontinuierlichen Abstände, z.B. jeden Tag, den Blutdruck und überträgt die Informationswerte. Diese werden jeweils mit den Vergleichswerten des Vergleichswertepaares V₁ verglichen und überprüft, ob der Informationswert innerhalb dieses Vergleichswerteintervalls liegt, und ob er insbesondere nicht den oberen Vergleichswert übersteigt, also noch weiter angestiegen ist. Ersichtlich nimmt im gezeigten Ausführungsbeispiel der Informationswert während der Einstellungsphase zwischen t₀ und t₁ kontinuierlich ab. Mit zunehmender Abnahme nähert sich der Informationswert dem unteren Vergleichswert des Vergleichswertepaares V₁. Ab einer bestimmten Annäherung - oder bei einem Unterschreiten - wird seitens der zentralen Systemsteuerungseinrichtung dem Informationswert I ein neues Vergleichswertepaar V₂ zugeordnet, bei dem der obere und der untere Vergleichswert um ein bestimmtes Maß abgesenkt wurden. Im Idealfall - und dieser wird von der ausgezogenen Kurve K₁ dargestellt - nimmt bei optimalem Therapieverlauf der Blutdruck kontinuierlich ab, bis er im Zeitpunkt t₁ einen Normalwert erreicht, der in der dem Zeitpunkt t₁ folgenden Einstellungsphase gehalten werden soll. Mit der kontinuierlichen Abnahme wird durch kontinuierlichen Vergleich des jeweils aufgenommenen Ist-Informationswert mit dem jeweiligen Vergleichswertepaar V₁ .... ₉ stets überprüft, ob der jeweilige Informationswert im zulässigen Bereich liegt. Falls sich dies bestätigt, wird gleichermaßen kontinuierlich überprüft, ob eine Anpassung des jeweiligen Vergleichswertepaares erforderlich ist und falls ja die nötige Änderung vorgenommen. D.h., das jeweilige Vergleichswertepaar wird dem jeweiligen Therapieverlauf derart angepasst, dass es stets mit dem Ist-Informationswert größenmäßig vernünftig korreliert ist.

Die Kurve K₁ zeigt den optimalen Therapieverlauf, d.h., es stellt sich eine kontinuierliche Verbesserung ein. Ergibt sich nun während des Therapieverlaufs die Situation, dass der Informationswert aus welchen Gründen auch immer sich nicht in Richtung des angestrebten Therapieziel ändert, sondern in unzulässiger Weise und unter Verschlechterung des Patientenzustandes ansteigt, wie dies durch die gestrichelte Kurve K₂ dargestellt ist, so tritt die Situation ein, dass der aufgenommene Informationswert aus dem definierten Vergleichswerteintervall herauswandert, d.h., es stellt sich eine unzulässige Abweichung des Informationswertes vom oberen Vergleichswert ein. Diese Situation ist in Fig. 1 mit dem mit A gekennzeichneten Punkt dargestellt. Bei durch den Vergleich des Informationswerts mit den Vergleichswerten erfasster unzulässiger Abweichung wird sofort eine Alarminformation an eine zuständige Person gegeben, die dieser mitteilt, dass bei dem überwachten Patienten eine deutliche, rapide und unzulässige Änderung des Zustandes vorliegt. Der behandelnde Arzt kann sofort entsprechende Maßnahmen ergreifen. Gleichzeitig erfolgt systemseitig eine erneute Anpassung des Vergleichswerteintervalls, das im gezeigten Beispiel deutlich angehoben wird in Anbetracht des stark gestiegenen Informationswerts und in Fig. 1 mit dem Vergleichswerteintervall V₃' dargestellt ist. Ersichtlich zeigt im gezeigten Beispiel die vom behandelnden Arzt sofort eingeleitete Therapiemaßnahme, z.B. Gabe stärkerer Medikamente oder anderer Medikamente oder eine andere Änderung der Behandlungsmethode, Wirkung, da die Kurve K₂ wieder deutlich abfällt. Der Informationswert I nähert sich wieder normalen Werten, wie sie der optimale Therapieverlauf hätte vermuten lassen. Anschließend stellen sich beim gezeigten Schema keine Schwierigkeiten mehr ein und der Informationswert, also der Blutdruckwert, sinkt kontinuierlich bis zum Erreichen des Therapieziels im Zeitpunkt t₁. Diesem Zeitpunkt ist das Vergleichswertepaar V₉ zugeordnet, welches auch in der Erhaltungsphase beibehalten wird. In dieser erfolgt zweckmäßigerweise bis zu einem bestimmten Zeitpunkt eine Nachkontrolle um sicherzustellen, dass der Blutdruckwert auch im zulässigen Normalbereich bleibt.

Wenngleich Fig. 1 lediglich die Berücksichtigung eines Informationswerts im Rahmen des erfindungsgemäßen Verfahrens beschreibt so ist es denkbar, zwei oder mehrere unterschiedliche Informationswerte im Rahmen des Vergleichs und der Anpassungsuntersuchungen und Auswertungen zu berücksichtigen. Das in Fig. 1 gezeigte Beispiel ist lediglich exemplarischer Natur.

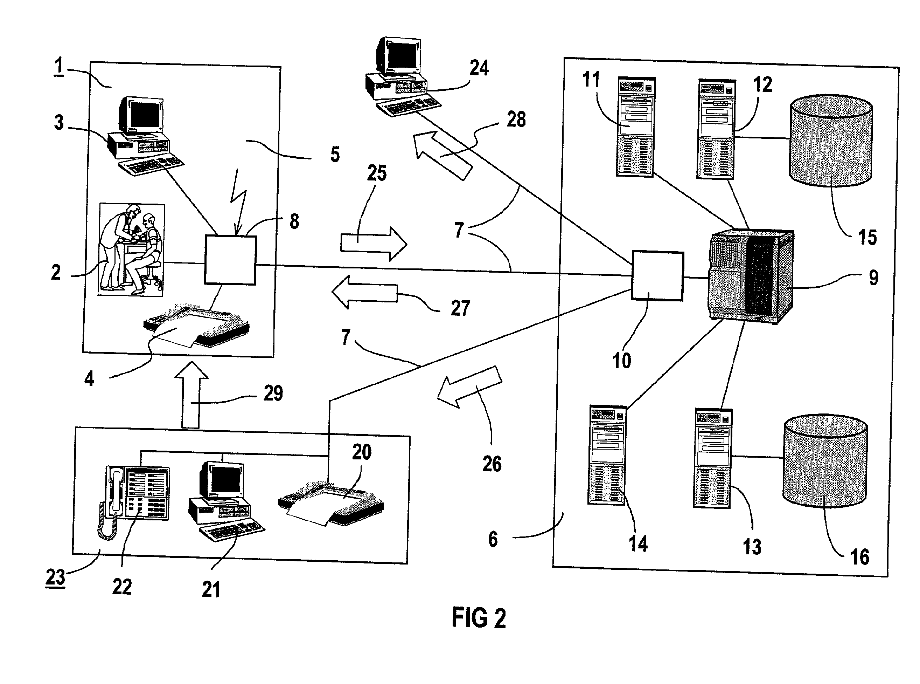

In der Fig. 2 ist ein erfindungsgemäßes System zur kontinuierlichen Überwachung medizinischer Parameter eines Patienten in seinem Haus 1 und damit zur Durchführung des erfindungsgemäßen Verfahrens dargestellt, in dem die Messwerterfassung 2 entweder automatisch oder manuell durch den Patienten- oder Informationswert oder eine Pflegeperson erfolgt. Diese Messoder Informationswerte können direkt per Personal Computer 3, per Faxgerät 4 oder per Telefon oder Handy 5 an eine Systemzentrale 6 weitergeleitet werden. Dies kann beispielsweise über ein ISDN-Netz 7 erfolgen, an das die entsprechenden Endgeräte über ein ISDN-Interface 8 angeschlossen sind.

In der Systemzentrale 6 ist ein Gateway 9 vorgesehen, das über ein ISDN-Interface 10 an dem ISDN-Netz 7 angeschlossen ist. An dem Gateway 9 kann ein Internet-Proxy-Server 11 zum Zugriff auf das Internet, eine Auswertevorrichtung 12 für die Mess- oder Informationswerte, ein Patientendaten-Server 13 zur Verwaltung der Patientendaten und ein Kommunikations-Server 14 als Routingvorrichtung zur Zusammenarbeit aller Komponenten und Weiterleitung von Meldungen angeschlossen sein. Mit der Auswertevorrichtung 12 ist ein Datenspeicher 15 für die Messwerte und mit dem Patientendaten-Server 13 eine Datenbank 16 als Speichervorrichtung verbunden.

Die Auswertevorrichtung 12 weist einen Komparator zum Vergleich der Mess- oder Informationswerte mit in dem Datenspeicher 15 gespeicherten Sollwerten und eine Alarmvorrichtung zur Erzeugung eines Alarmsignals bei Überschreitung der Messwerte von gespeicherten Vergleichsgrenzen auf. Sie nimmt erforderlichenfalls die Erzeugung etwaiger Rechenwerte, etwaiger Trendanalysen sowie gegebenenfalls auch die Anpassung der Vergleichswerte vor.

An der Systemzentrale 6 sind Empfangsgeräte wie beispielsweise ein Faxgerät 20, Personal Computer 21 oder ein Telefon 22 oder Handy angeschlossen, die zu einer Ärzte-Bereitschaft gehören und beispielsweise in einer Gemeinschaftspraxis 23 von Ärzten angeordnet sein kann.

Weiterhin ist ein Personal Computer 24 einer Praxis eines erstbehandelnden Arztes über das ISDN-Netz 7 mit dem Gateway 9 der Systemzentrale 6 verbunden.

In der Systemzentrale 6 werden die medizinischen Daten über genormte Telekommunikationsschnittstellen eingelesen und in dem Datenspeicher 15 langfristig abgespeichert, die Auswertevorrichtung 12 wertet die medizinischen Daten und den Zeitpunkt der Datenübertragung aus, um Informationsnachrichten versenden zu können. Die Datenübertragung erfolgt beispielsweise über das Internet oder Telefonnetz. Die Vergabe einer Patientenidentifikationsnummer erfolgt über eine Sicherheitsarchitektur.

In der Systemzentrale 6 können alle Informationen zusammengeführt werden, die zur Realisierung der speziellen Ausführungen des Alarmgebers und zur Realisierung einer Weiterleitung von Nachrichten bei Abwesenheit des Empfängers benötigt werden.

Die Endgeräte 2 bis 5 für den Patienten dienen zur Erfassung der medizinischen Daten beim Patienten und Übertragung der Daten an die Systemzentrale 6. Es können von der Messtechnik beliebige existierende Geräte verwendet werden, beispielsweise EKG-, Blutdruck-, Blutzuckermessgeräte, Teststreifen, Peak-Flowmeter und/oder Vitalkapazitätsmessgeräte. Die Datenübergabe 25 der Mess- oder Informationswerte an den Server in der Systemzentrale 6 kann durch das Messgerät selbst erfolgen, oder über unten beschriebene Eingabemöglichkeiten realisiert werden.

Für die Pfleger ist ein Personal Digital Assistant (PDA) oder ein Laptop vorgesehen, in dem alle Patientendaten von Patienten eingegeben werden, die von diesem Pflegedienst besucht werden. Die Synchronisierung der Daten kann sofort, beispielsweise per Handy-Internet Verbindung, oder zu einem späteren Zeitpunkt erfolgen, wenn die Schwester wieder im Büro oder Praxis eintrifft.

Die Systemzentrale 6 dient zur Entgegennahme medizinischer Daten über eine der Telekommunikationsschnittstellen und Speicherung in dem Datenspeicher 15. Weiterhin soll sie eine Weiterverarbeitung der medizinischen Daten mit Hilfe der Auswertevorrichtung 12 für die Mess- oder Informationswerte mit der Alarmvorrichtung und dem Datenspeicher 15 bewirken. Eine Ausgabe der im Datenspeicher 15 gewonnenen Daten an eines der Endgeräte für Ärzte erfolgt über eine Telekommunikationsschnittstelle.

Ein Alarm wird durch die Alarmvorrichtung in der Auswertevorrichtung 12 bei Vorliegen einer der Ereigniskonstellationen wie das Ausbleiben von Mess- oder Informationswerten, krankhafte Mess- oder Informationswerte oder Ausbleiben der Reaktion auf generierte Alarme ausgelöst. Dabei wird ein Alarm 26 aufgrund des Abweichens der Mess- oder Informationswerte von den Vergleichswerten an die Endgeräte 20 bis 22 der Gemeinschaftspraxis 23 weitergeleitet. Ein Alarm 27 an den Patienten wird wegen fehlender Mess- oder Informationswerte ausgelöst. In der Praxis kann der Arzt die in dem Datenspeicher 15 gespeicherten Mess- oder Informationswerte 28 mit dem Personal Computer 24 abgerufen werden.

Die Auswertevorrichtung 12 beurteilt, ob Mess- oder Informationswerte oder Wertkonstellationen als krankhaft einzustufen sind. Als Kriterium werden dabei Vergleichsgrenzen aus der Literatur entnommenen, die in dem Datenspeicher 15 abgespeichert sind. Weiterhin können vom behandelnden Arzt für den Patienten individuelle Grenzen definiert werden. Die Vergleichswerte sind variierbar und können dem Therapieverlauf angepasst werden.

Dazu kann ferner ein Expertensystem eingesetzt werden, das die Werte krankheits- und problemspezifisch anhand von Regelsystemen oder auf Wahrscheinlichkeiten basierend interpretiert. Dabei ist eine Individualisierung des Expertensystems auf den Patienten vorsehbar, d.h. das Expertensystem lernt den Patienten, den es überwacht, im Überwachungsprozess immer besser kennen, es trifft dazu als lernendes System ständig Prognosen über zu erwartende zukünftige Mess- oder Informationswerte, die es mit den wahren Werten vergleicht. Damit wird eine individualisierte Überwachung realisiert.

Im Fall komplexer Alarme kann ein Expertensystem eingesetzt werden, dass die Prozesskette, Erinnern des Patienten, Verständigung Hausarzt, Rufen des Notarztes, Organisieren des Transportdienstes und/oder Vorbereitung der Klinik unter Berücksichtigung der Verfügbarkeit der Alarmempfänger übernimmt.

Die Prozesskette ist dabei die Gesamtheit der beteiligten Personen und Institutionen. Dazu gehören der Patient, die Behandlungskette mit Arzt, Pflege- und Transportdienst sowie die Notfallkette mit Feuerwehrleitstelle, Notfall- und Transportdienste sowie das Krankenhaus.

Alarme können in Abhängigkeit von der Dringlichkeit einer Reaktion in unterschiedlichen Dringlichkeitsstufen generiert werden, beispielsweise dringendst, dringend, Routine oder Standard. Welche Messwerte zu welchen Dringlichkeitsstufen führen, kann mit den gleichen Mechanismen wie die Beurteilung der Messwerte festgelegt werden und in dem Datenspeicher 15 abgespeichert sein.

Die Alarm-Endgeräte dienen der Ausgabe der vom Nachrichtenversand versandten Nachrichten beim Empfänger. Hierzu können spezielle Ausführung des Endgeräts für Patient und/oder Pfleger dienen wie PDA mit Modem, Telefon mit Sprache oder Tonwahl (DTMF), Messgerät mit integriertem Modem, TV Set-Top-Box, Handy, WWW-Formular oder Papierformular und WWW-Formular.

Das Alarm-Endgerät kann je nach Alarmstufe beispielsweise auch ein Telefon, Handy, E-Mail-Terminal, WWW-Terminal, Fax-Gerät, Terminal zur Nutzung eines proprietären Diensts oder auch der Briefkasten sein.

Die Systemzentrale 6 besitzt eine Schnittstelle zur Kopplung mit einem QS-System, um eine Ergebnisqualität unter reallife-Bedingungen zu erhalten. Weiterhin kann das zentrale System eine Schnittstelle zur Kopplung mit einem Abrechnungsmodul aufweisen.

Spezielle Ausführungen der Alarmvorrichtung können eine Alarmauslösung bei pathologischen oder fehlerhaft erfassten Mess- oder Informationswerten, eine Alarmauslösung bei Compliance-Problemen des Patienten, eine Alarmauslösung bei zu langer Reaktionszeit des Arztes auf dringende Nachrichten und eine Alarmweiterleitung sein, falls Arzt nicht reagiert.

Nachrichten können beispielsweise über Sprachausgabe per Telefon, SMS, E-Mail, WWW, WAP, Fax, Proprietärer Dienst oder Briefpost übermittelt werden.

Die Auswertevorrichtung 12 für die Messwerte und der Patientendaten-Server 13 können zur Datenerhebung so ausgeführt sein, dass die medizinischen Daten sicher und pseudonymisiert gespeichert werden.

An der Systemzentrale 6 sind Empfangsgeräte wie beispielsweise ein Faxgerät 20, Personal Computer 21 oder ein Telefon 22 oder Handy angeschlossen, die zu einer Ärzte-Bereitschaft gehören und beispielsweise in einer Gemeinschaftspraxis 23 von Ärzten angeordnet sind.

Das Alarm-Endgerät in der Gemeinschaftspraxis 23 kann einen Rückkanal 29 zur Übertragung von Therapie-Hinweisen an den Patient und/oder Pfleger aufweisen, so dass eine Therapie trotz räumlicher Trennung von Arzt und Patient durchgeführt werden kann (Teletherapie).

## Patentansprüche

1. Verfahren zum Überwachen des Therapieverlaufs eines zu therapierenden Patienten, bei dem wenigstens ein eine therapeutische und/oder diagnostische Maßnahme erfordernder Informationswert, der ein direktes oder indirektes Maß für den Therapieverlauf ist, kontinuierlich oder in Zeitintervallen aufgenommen wird, und dem wenigstens ein mit ihm oder mit einem basierend auf dem Informationswert ermittelten Rechenwert zu vergleichender Vergleichwert zugeordnet ist, wobei bei einer im Rahmen des Vergleichs festgestellten gegebenen oder sich einstellenden unzulässigen Abweichung des Informationswerts oder des Rechenwerts vom Vergleichswert eine Alarminformation ausgegeben wird, und wobei der Vergleichswert in Abhängigkeit des Therapieverlaufs variiert und dem Therapieverlauf angepasst wird, **dadurch gekennzeichnet, dass** die Anpassung des wenigstens einen Vergleichswerts automatisch oder durch eine autorisierte Person oder Stelle erfolgt, wobei die Anpassung in vorgegebenen Zeitabständen oder in variablen Zeitabständen und abhängig vom aufgenommenen Informationswert oder dem ermittelten Rechenwert erfolgt, und wobei bei einer durch eine Person oder Stelle erfolgenden Anpassung die Person oder Stelle automatisch auf das Erfordernis der Anpassung hingewiesen wird.

2. Verfahren nach Anspruch 1, **dadurch gekennzeichnet, dass** dem Informationswert oder dem Rechenwert ein oberer und ein unterer Vergleichswert zugeordnet werden, wobei der Informationswert oder der Rechenwert im Rahmen des Vergleichs mit beiden verglichen wird.

3. Verfahren nach Anspruch 2, **dadurch gekennzeichnet, dass** eine unzulässige Abweichung dann gegeben ist, wenn der Informationswert oder der Rechenwert oberhalb des oberen oder unterhalb des unteren Vergleichswerts ist.

4. Verfahren nach einem der vorangehenden Ansprüche, **dadurch gekennzeichnet, dass** eine sich einstellende unzulässige Abweichung anhand eines Vergleichs eines sich durch Analyse zeitlich früher aufgenommener Informationswerte oder ermittelter Rechenwerte ermittelten Therapietrends mit dem wenigstens einen Vergleichswert erfolgt.

5. Verfahren nach einem der vorangehenden Ansprüche, **dadurch gekennzeichnet, dass** die Anpassung in Abhängigkeit mehrerer unterschiedlicher aufgenommener Informationswerte oder ermittelter Rechenwerte erfolgt, denen jeweils wenigstens ein Vergleichswert zugeordnet ist.

6. Verfahren nach einem der vorangehenden Ansprüche, **dadurch gekennzeichnet, dass** die Anpassung in Abhängigkeit des Vergleichs des Informationswerts oder des Rechenwerts mit dem wenigstens einen Vergleichswert erfolgt, derart, dass bei einem zulässigen Abweichen von dem Vergleichswert die Anpassung vorgenommen wird.

7. Verfahren nach einem der Ansprüche 1 bis 5, **dadurch gekennzeichnet, dass** die Anpassung in Abhängigkeit eines aus der Aufnahme zeitlich früherer Informationswerte oder ermittelter Rechenwerte ermittelten Therapietrends erfolgt.

8. Verfahren nach einem der vorangehenden Ansprüche, **dadurch gekennzeichnet, dass** jedem Vergleichswert ein Toleranzwertebereich zugeordnet ist.

9. Verfahren nach einem der vorangehenden Ansprüche, **dadurch gekennzeichnet, dass** der verantwortlichen Person oder Stelle eine Information gegeben wird, wenn sich innerhalb eines vorbestimmten Zeitintervalls ein angestrebter, durch die Aufnahme des Informationswerts oder die Ermittlung des Rechenwerts erfassbarer Therapieerfolg nicht einstellt.

10. Verfahren nach Anspruch 9, **dadurch gekennzeichnet, dass** wenigstens einem Vergleichswert wenigstens ein Zeitintervallwert zugeordnet ist, wobei die Information ausgegeben wird, wenn der Informationswert oder der Rechenwert innerhalb des mittels des Zeitintervallwerts definierten Zeitintervalls keine zulässige Abweichung vom Vergleichswert aufweist.

11. Verfahren nach Anspruch einem der vorangehenden Ansprüche, **dadurch gekennzeichnet, dass** der Person oder Stelle während der Überwachung, insbesondere mit der Gabe der Information über die Nichterreichung des Therapieziels weitere therapeutisch oder diagnostisch relevante Informationen gegeben werden.

12. Verfahren nach Anspruch 11, **dadurch ge-kennzeichnet, dass** die weiteren relevanten Informationen aus einer Wissensdatenbank gelesen werden.

13. Verfahren nach einem der vorangehenden Ansprüche, **dadurch gekennzeichnet, dass** sämtliche Schritte unter Verwendung eines Expertensystems erfolgen.

14. Verfahren nach einem der vorangehenden Ansprüche, **dadurch gekennzeichnet, dass** der Informationswert mittels eines Erfassungsgeräts aufgenommen und automatisch an eine Auswerteeinrichtung übermittelt wird, oder dass der Informationswert vom Patienten gegebenenfalls unter Verwendung eines Erfassungsgeräts ermittelt und in ein Übermittlungsgerät zum Übermitteln an eine Auswerteeinrichtung eingegeben wird.

15. Verfahren nach Anspruch 14, **dadurch gekennzeichnet, dass** eine zentrale, externe Auswerteeinrichtung verwendet wird, die in Abhängigkeit der Auswertung des Informationswerts die Alarminformation automatisch an wenigstens ein Alarminformationsempfangsgerät übermittelt.

16. System zum Überwachen des Therapieverlaufs eines zu therapierenden Patienten, geeignet zur Durchführung des Verfahrens nach einem der Ansprüche 1 bis 15.
